# EUROPÄISCHE PATENTSCHRIFT

(11) **EP 0 425 893 B1**
(45) Veröffentlichungstag und Bekanntmachung des Hinweises auf die Patenterteilung: **16.06.1993**
(21) Anmeldenummer: 90119880.4
(22) Anmeldetag: 17.10.1990
(51) Int. Cl.: C07C 43/13, C07C 41/34, B01D 61/36

(54) **Verfahren zur Trennung eines Gemisches aus 1-Methoxypropanol-2 und Wasser in seine Bestandteile**
Process for the separation of a mixture of 1-methoxy-2-propanol and water into its components
Procédé de séparation d'un mélange de 1-méthoxypropanol-2 et d'eau dans ses composants

(30) Priorität: 28.10.1989 DE 3936052
(43) Veröffentlichungstag der Anmeldung: 08.05.1991
(73) Patentinhaber: BASF Aktiengesellschaft, 67063 Ludwigshafen (DE)
(72) Erfinder: Krug, Joseph, Dr., W-6700 Ludwigshafen (DE); Reissenweber, Gernot, Dr., W-6737 Boehl-Iggelheim (DE); Koob, Knut, Dr., W-6704 Mutterstadt (DE)

(56) Entgegenhaltungen:
- EP-A- 0 307 636
- US-A- 4 728 429
- DESALINATION, Band 53, 1985, Seiten 327-338, Elsevier Science Publishers B.V., Amsterdam, NL; G.F. TUSEL et al.: "Use of pervaporation systems in the chemical industry"

## Beschreibung

Die Erfindung betrifft ein Verfahren zur Trennung eines Gemisches aus 1-Methoxypropanol-2 und Wasser in seine Bestandteile.

1-Methoxypropanol-2 ist ein häufig verwendetes Lösungsmittel in der chemischen Industrie, beispielsweise als Lösungsmittel für chemische Reaktionen oder für die Umkristallisation von bestimmten Verbindungen. Nach dem Einsatz fällt dabei das 1-Methoxypropanol-2 oft wasserhaltig an.

Da sich das Gemisch beispielsweise durch einfache Destillation/Rektifikation nur bis zum Azeotrop auftrennen läßt und die üblichen Methoden zur Stofftrennung von Azeotropen wie beispielsweise Druckwechselverfahren, Schleppmitteldestillation etc. nicht zum Erfolg führen, fallen bei der Gewinnung des 1-Methoxypropanol-2 zwangsläufig hohe Verluste an. Das Azeotrop selbst mußte bisher aufwendig entsorgt werden, beispielsweise durch Verbrennung.

Der Erfindung liegt daher die Aufgabe zugrunde, Verluste des Wertproduktes 1-Methoxypropanol-2 zu vermeiden.

Diese Aufgabe wird erfindungsgemäß dadurch gelöst, daß die Trennung mittels Pervaporation durchgeführt wird, wobei das Gemisch flüssig oder gasförmig über eine hydrophile Membrane geführt wird.

Das Verfahren kann technisch sowohl in Batch - als auch in kontinuierlicher Fahrwelse betrieben werden. Beide Verfahrensmöglichkeiten werden nachstehend detailliert beschrieben.

### Beispiel 1

### Batchfahrweise, Entwässerung von 1-Methoxypropanol-2 durch Pervaporation

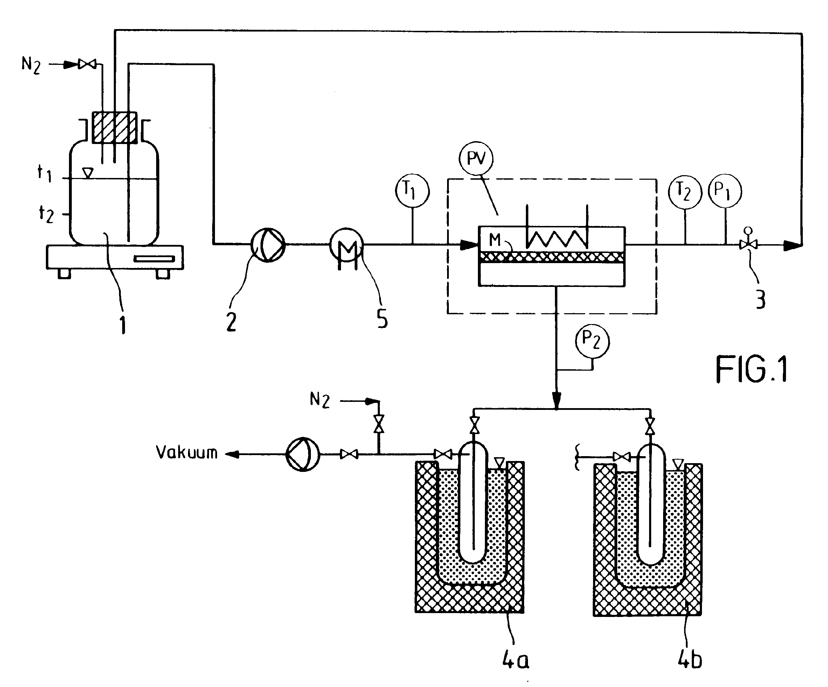

Das diskontinuierliche durchgeführte Verfahren ist in Figur 1 dargestellt. Dabei wird ein Gemisch aus 55 Gew.-% 1-Methoxypropanol-2 und 45 Gew.-% Wasser mit 718 gr im Behälter 1 bei Raumtemperatur vorgelegt. Diese Vorlaufmenge wird über die Pumpe 2 und die Testzelle PV mit der Membranfläche M von 100 cm² ca. 7 mal in der Stunde im Kreis gefahren. Am Druckhalteventil 3 wird ein Überdruck von 2,5 bar eingestellt. Auf der Permeatseite liegt ein absoluter Druck von 25 mbar an. Das Permeat wird in den Kühlfallen 4a oder 4b bei ca. -80°C ausgefroren. Die Analyse des Permeats und des Behälterinhaltes erfolgt ca. 1 mal in der Stunde. Nach der An-Einfahrweise wird das Feed vom Thermostaten 5 innerhalb einer Stunde von Raumtemperatur auf 95°C erwärmt und konstant gehalten (Zeitpunkt t₁). Der Temperaturunterschied zwischen der Feedtemperatur T₁ (PV-Ein) und der Retentattemperatur T₂ (PV-Aus) beträgt dann im Mittel ca. 2,8°C. Nach 11 Stunden (Zeitpunkt t₂) ergibt sich eine Endproduktmenge von 409 gr mit 96 Gew.-% 1-Methoxypropanol-2 und 4 Gew.-% Wasser. Durch die hydrophile Membran sind 309 gr mit 98 Gew.-% Wasser und 2 Gew.-% 1-Methoxypropanol-2 permeiert. Der Fluß hat dabei von ca. 4,6 kg/m²h auf 1,2 kg/m²h abgenommen.

### Beispiel 2

### Kontinuierliche Fahrweise, Entwässerung von 1-Methoxypropanol-2 durch Pervaporation

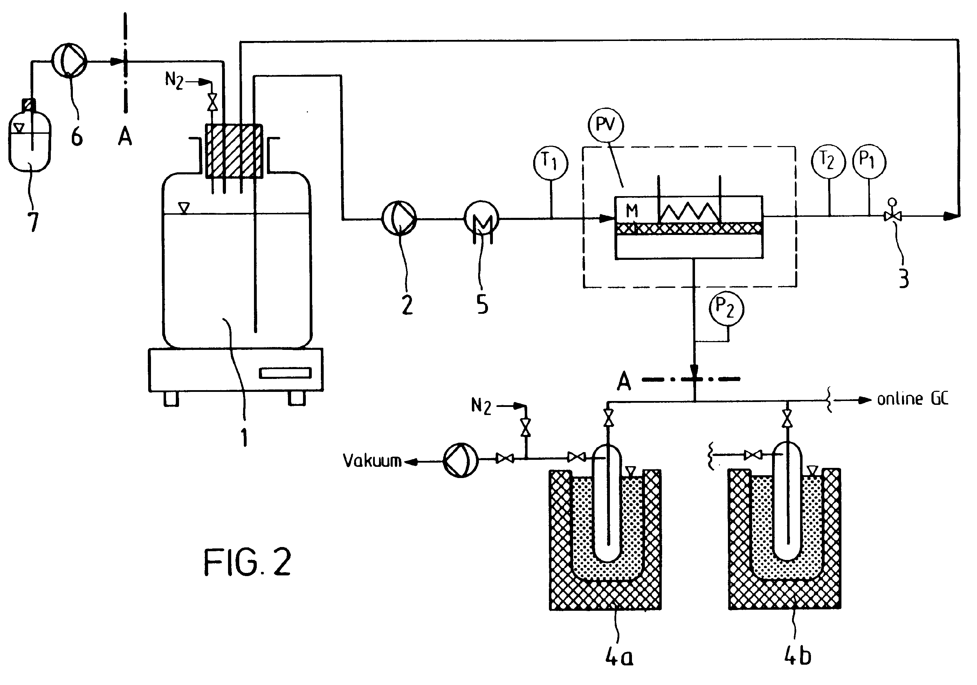

Das kontinuierlich durchgeführte Verfahren ist in der Figur 2 dargestellt. Dabei wird ein Gemisch aus 86,8 Gew.-% 1-Methoxypropanol-2 und 13,2 Gew.-% Wasser mit ca. 10 kg im Behälter 1 bei Raumtemperatur vorgelegt. Die Pumpe 2 fördert ca. 5 kg/h im Kreis. Die Membranfläche M der Pervaporation beträgt 100 cm². Am Druckhalteventil 3 wird ein Überdruck von 2,5 bar eingestellt. Auf der Permeatseite liegt ein absoluter Druck von 52 mbar an. Das Permeat wird entweder in den Kühlfallen 4a und 4b bei ca. -80°C ausgefroren oder im GC-Onlinebetrieb analysiert. Nach der Anfahrweise wird das Feed vom Thermostaten 5 innerhalb einer Stunde von Raumtemperatur auf 95°C erwärmt und konstant gehalten. Der Temperaturunterschied zwischen der Feedtemperatur T₁ (PV-Ein) und der Retentattemperatur T₂ (PV-Aus) beträgt dann ca. 5,9°C. Über eine Dosierpumpe 6 wird aus der Vorlage 7 dem Behälter 1 ungefähr soviel Gemisch zugeführt, wie Permeat bei der Pervaporation anfällt (Schnitt A-A). Das Permeat besteht aus 98,9 Gew.-% Wasser und 1,1 Gew.-% 1-Methoxypropanol-2. Der Fluß beträgt ca. 1,5 kg/m²h. Diese stationäre Betriebsweise mit konstanter Feedzusammensetzung wurde über einen Zeitraum von ca. 21 Stunden gefahren.

Die mit der Erfindung erzielten Vorteile bestehen darin, daß durch die Aufarbeitung des Azeotrops das Lösungsmittel 1-Methoxypropanol-2 zurückgewonnen und damit in den Prozeß zurückgeführt werden kann. Außerdem entfallen zusätzlich die Kosten für die Verbrennung des azeotropen Gemisches.

## Patentansprüche

1. Verfahren zur Trennung eines Gemisches aus 1-Methoxypropanol-2 und Wasser in seine Bestandteile, dadurch gekennzeichnet, daß die Trennung mittels Pervaporation durchgeführt wird.

2. Verfahren gemäß Anspruch 1, dadurch gekennzeichnet, daß die Trennung mittels einer hydrophilen Membrane durchgeführt wird.

## Claims

1. A process for separating 1-methoxy-2-propanol and water by pervaporation.

2. A process as claimed in claim 1, wherein a hydrophilic membrane is used.

## Revendications

1. Procédé pour séparer un mélange de 1-méthoxypropanol-2 et d'eau en ses composants, caractérisé par le fait que l'on effectue la séparation par pervaporation.

2. Procédé selon la revendication 1, caractérisé par le fait que la séparation est effectuée au moyen d'une membrane hydrophile.
